# EUROPÄISCHE PATENTSCHRIFT

(11) **EP 1 558 775 B1**
(45) Veröffentlichungstag und Bekanntmachung des Hinweises auf die Patenterteilung: **25.04.2007**
(21) Anmeldenummer: 03782187.3
(22) Anmeldetag: 07.11.2003
(51) Int. Cl.: C22C 19/07, A61K 6/04, A61C 13/00

(54) **DENTALGUSS-LEGIERUNG**
DENTAL CASTING ALLOY
ALLIAGE POUR PIECE DENTAIRE COULEE

(30) Priorität: 07.11.2002 DE 10252776
(43) Veröffentlichungstag der Anmeldung: 03.08.2005
(73) Patentinhaber: DENTAURUM J.P. WINKELSTROETER KG, 75228 Ispringen (DE)
(72) Erfinder: LINDIGKEIT, Jürgen, 75203 Königsbach-Stein (DE)
(74) Vertreter: Hoeger, Stellrecht & Partner Patentanwälte
(86) Internationale Anmeldenummer: PCT/EP2003/012465
(87) Internationale Veröffentlichungsnummer: WO 2004/042098

(56) Entgegenhaltungen:
- DE-A- 3 941 820
- DE-A- 19 714 034
- DE-C- 3 415 249
- DE-C- 19 845 638
- FR-A- 2 733 416
- US-A- 4 459 263

## Beschreibung

Die Erfindung betrifft eine Dentalguss-Legierung für die Herstellung von dentalen Prothesengerüsten.

Gusslegierungen für dentale Prothesengerüste, insbesondere für sogenannte Modellgussprothesen, sind bereits seit dem Jahre 1935 bekannt.

Solche Modellgusslegierungen enthalten neben Chrom und Molybdän hohe Gehalte an Kohlenstoff, um die entsprechende, zahntechnisch erforderliche Starrheit und eine leichtflüssige Schmelze zu erzielen, die das präzise Abgießen der Modelle erleichtert.

Mangan und Silizium wird bei diesen Legierungen eingesetzt, um die Fließfähigkeit entsprechend positiv zu beeinflussen.

Aus der DE 36 09 184 C2 ist die Verwendung einer Legierung zur Herstellung von Gussteilen für die Dentaltechnik bekannt. Diese Legierung weist Molybdängehalte von 4,5 bis 5,5 Gew.% auf.

Aus der DE 198 15 091 C2 ist eine Legierung für Dentalgussteile bekannt, welche einen Molybdängehalt von 4 bis 8 Gew.% verlangt. Darüber hinaus soll ein Anteil von 0,05 bis 1,2 Gew.% Tantal, Niob und/oder Wolfram vorhanden sein, wobei der Anteil jedes einzelnen der Elemente Tantal, Niob oder Wolfram weniger als 0,5 Gew.% beträgt.

An Legierungen, die in der Dentaltechnik verwendet werden sollen, werden im Allgemeinen besondere Anforderungen gestellt. Beispielsweise müssen Aufbrennlegierungen für die Metallkeramik mit den handelsüblichen Dentalkeramiken hinsichtlich der thermischen Expansion und Kontraktion kompatibel sein. Außerdem müssen diese Legierungen eine die Haftung zwischen Metall und Keramik gewährleistende Oxidschicht geringer Dicke aufbauen. Auch darf die Oxidfarbe aus ästhetischen Gründen nicht durch die opake Keramik hindurch scheinen. Bei nicht zu verblendenden Dental-Gussstücken, z.B. bei herausnehmbaren Prothesen mit Klammern, wird dazu eine gewisse Aktivierbarkeit und Federhärte verlangt. Besonders wichtig ist in der Dentaltechnik darüber hinaus, dass die Verarbeitung der verwendeten Legierungen mit im Dentallabor zur Verfügung stehenden Mitteln erfolgen kann, sie sollten also mit den üblichen Gussschleudern vergossen werden können. Deshalb enthalten die Legierungen, die in der Dentallegierung häufig verwendet werden, bei Anwendung als Modellgusswerkstoff einen weit höheren Kohlenstoffgehalt, als es nach verschiedenen Normen zulässig ist. Darüber hinaus sind Dentalguss-Legierungen zu bevorzugen, deren Härte im gegossenen Zustand nicht allzu sehr von der Härte des natürlichen Zahnschmelzes abweicht, so dass bei Kontakt von Dentalguss-Legierung und Zahnoberfläche ein nennenswerter abrasiver Verschleiß des Zahnes vermieden wird. Weiterhin ist es vorteilhaft, wenn die Legierung mit geringen Nickelgehalten hergestellt werden kann, da dann auch Nickel-allergische Patienten mit solche Prothesen versorgt werden können.

Aufgabe der vorliegenden Erfindung ist es, unter Beachtung der vorstehend genannten Beschränkungen eine Gusslegierung der eingangs beschriebenen Art vorzuschlagen, welche einen hohen Wert bei der Bruchdehnung aufweist und keine übergroße Vickers-Härte (HV 10) zeigt.

Diese Aufgaben werden bei der eingangs beschriebenen Dentalguss-Legierung erfindungsgemäß dadurch gelöst, dass diese besteht aus

| | | |
|---|---|---|
| 25 - 32 | Gew.% | Chrom |
| 8 - 12 | Gew.% | Wolfram, |

einem oder mehreren Elementen der 4. und/oder 5. Nebengruppe des Periodensystems der Elemente mit jeweils einem Anteil von 0,05 - 0,4 Gew.%, Rest Kobalt.

Optional beinhalten erfindungsgemäße Legierungen ferner

| | | |
|---|---|---|
| 0 bis 1 | Gew.% | Mn |
| 0,8 bis 1,6 | Gew.% | Si und/oder |
| 0,1 bis 0,35 | Gew.% | N. |

Daneben kommen herstellungsbedingte Verunreinigungen von jeweils weniger als 0,1 Gew.% vor.

Bei einer bevorzugten erfindungsgemäßen Dentalgusslegierung beträgt der Gesamtgehalt der Elemente der 4. und/oder der 5. Nebengruppe des Periodensystems der Elemente ca. 0,05 bis ca. 0,4 Gew.%.

Ferner ist bevorzugt, wenn die Elemente der 4. und 5. Nebengruppe des Periodensystems der Elemente ausgewählt sind aus Titan, Zirkon, Niob und Tantal.

Ferner sind erfindungsgemäße Legierungen bevorzugt, die im Wesentlichen frei sind von den Elementen Eisen, Vanadium, Nickel, Molybdän und/oder Kohlenstoff.

Der Zusatz von Mn wirkt in der Schmelze desoxidierend und bindet Sauerstoff unter Schlackenbildung.

Der Siliziumanteil dient der Absenkung der Viskosität der Schmelze, so dass sehr feine Details im Guss gut ausgebildet werden.

Der Stickstoffanteil erhöht die Duktilität. Bei zu geringen Anteilen erhält man eine schlechtere Bruchdehnung und geringere Streckgrenze.

Mit den erfindungsgemäßen Dentalgusslegierungen können prothetische Gerüste mit hoher Korrosionsbeständigkeit hergestellt werden, die darüber hinaus gute Verarbeitungseigenschaften, insbesondere eine niedrige Härte und einen niederen Wärmeausdehnungskoeffizienten sowie eine gute Laserschweißbarkeit aufweisen.

Die niedrigen Wärmeausdehnungskoeffizienten vereinfachen die Verblendung der Gerüste mit Keramik.

Im Folgenden sei eine konkrete, beispielhafte Zusammensetzung der erfindungsgemäßen Legierung angegeben (Tabelle 1), wobei betont werden darf, dass sich die Erfindung selbstverständlich nicht auf diese konkret angegebene Legierung beschränkt. In der folgenden Tabelle 2 sind die mechanischen Eigenschaften der Legierung gemäß Tabelle 1 zusammengefasst.

Tabelle 3 fasst Vergleichsbeispiele 1 bis 6 zusammen und zeigt gegenüber dem obigen Beispiel einer erfindungsgemäßen Legierung die Bedeutung der Einhaltung der Grenzen in der Elementzusammensetzung, um ausgewogene, gute Eigenschaften der Dentalguss-Legierung zu erhalten.

**Tabelle 1**

| **Elementzusammensetzung** | **Gewichtsprozent (Gew.%)** |
|---|---|
| | |
| Co | 60 |
| Cr | 28 |
| Mo | - |
| W | 9,8 |
| Mn | 0,3 |
| Si | 1,5 |
| N | 0,2 |
| Nb | 0,2 |

**Tabelle 2**

| **Mechanisch/physikalische Eigenschaften** | |
|---|---|
| | |
| Streckgrenze Rₚ 0,2 (MPa) | 620 |
| Zugfestigkeit Rₘ (MPa) | 845 |
| E-Modul (GPa) | 190 |
| Bruchdehnung A₅ (%) | 10,2 |
| Vickers-Härte HV 10 | 300 |
| Wärmeausdehnungskoeffizient (WAK) im Bereich von 20 - 500 °C (10⁻⁶ K⁻¹) | 14,1 |

**Tabelle 3**

| | Vergleichsbeispiele | | | | | |
|---|---|---|---|---|---|---|
| | #1 | #2 | #3 | #4 | #5 | #6 |
| Co | 70 | 68 | 59,5 | 68 | 61 | 65 |
| Cr | 20 | 20 | 20 | 20 | 24 | 28 |
| Mo | 4,5 | 5 | 5 | - | 7 | 4,5 |
| W | 5 | 5 | 5 | 10 | 5 | - |
| Mn | 0,3 | 0,3 | 0,3 | 0,3 | 0,25 | 0,25 |
| Si | 0,1 | 1,5 | - | 1,5 | 1,5 | 1,6 |
| N | 0,1 | 0,2 | 0,2 | 0,2 | 0,25 | 0,2 |
| Fe | - | - | 10 | - | - | 0,35 |
| sonstige | | | | | Ce, C | Al, La, Ce |
| HV 10 | 289 | 297 | 280 | 291 | 340 | 310 |
| WAK (10⁻⁶ K⁻¹) | 15,05 | 14,95 | 15,43 | 15,4 | 14 | 14,7 |
| R_{P} 0,2 (MPa) | 380 | 480 | 467 | 457 | 700 | 520 |
| Rₘ (MPa) | 630 | 636 | 698 | 675 | 900 | 730 |
| A₅ (%) | 17,5 | 10,5 | 18,1 | 15,7 | 7 | 11 |

Die vorstehend in der Tabelle der Vergleichsbeispiele zusammengefassten Legierungen sind zwar alle als Gerüstwerkstoff und zum keramischen Verblenden geeignet, haben aber alle den einen oder anderen oder auch mehrere Nachteile im Vergleich zu der erfindungsgemäßen Legierung.

Die Legierung gemäß Vergleichsbeispiel 5 hat zwar eine niedrige Wärmeausdehnung, jedoch eine relativ hohe Härte.

In Vergleichsbeispiel 6 weist die Legierung zwar mit 310 HV 10 eine relativ geringe Härte auf, hat aber den Nachteil eines hohen Wärmeausdehnungskoeffizienten, was unter Umständen Schwierigkeiten bei der Verblendung mit Keramik mit sich bringen kann.

Die Vergleichslegierungen 1 bis 4 weisen zwar eine geringe Härte auf, weisen aber weitaus höhere Wärmeausdehnungskoeffizienten auf als die erfindungsgemäße Legierung und sind deshalb schwierig mit Keramik zu verarbeiten.

Dies gilt auch für die molybdänfreie Vergleichslegierung 4, der gegenüber die erfindungsgemäße Legierung eine niedrigere Wärmeausdehnung aufweist und trotz des hohen Wolframgehalts überraschenderweise eine geringe Härte und eine erstaunlich hohe Bruchdehnung von über 10 % aufweist.

Der erfindungsgemäße Zusatz von einem Element der 4. oder 5. Nebengruppe, insbesondere ausgewählt aus den Elementen Titan, Zirkon, Niob und Tantal, verbessert die Korrosionsbeständigkeit und erhöht die Warmfestigkeit beim Aufbrennen der keramischen Massen.

Das Element Niob ist zudem vergleichbar biokompatibel, wie Titan oder Tantal oder Zirkon, und ruft keine Gewebereaktionen hervor.

Die erfindungsgemäße Legierung ist demnach auch besonders bioverträglich.

Die bevorzugten Elemente der 4. und 5. Nebengruppe des Periodensystems der Elemente, nämlich Niob, Titan, Tantal und Zirkon, binden auch den Restkohlenstoff in der Legierung ab und verhindern so die Bildung von Chromcarbiden. Dies wiederum bewirkt eine gute Laserschweißbarkeit der erfindungsgemäßen Legierung.

Das Vorhandensein der Elemente Niob, Titan, Tantal und/oder Zirkon ist auch verantwortlich für eine starke Passivierung der erfindungsgemäße Legierung. Daraus resultiert eine besonders hohe Korrosionsbeständigkeit.

Hervorzuheben ist schließlich, dass die erfindungsgemäße Legierung keine Edelmetallanteile enthält, wie sie bei einigen herkömmlichen Legierungen mit niedriger Härte verwendet werden und die zum Teil bis zu 2 % Gold enthalten, um die Härte der Legierung auf einen verträglichen Wert zu reduzieren.

## Patentansprüche

1. Dentalguss-Legierung, insbesondere für die Herstellung von dentalen Prothesengerüsten, bestehend aus
| | | |
|---|---|---|
| 25 - 32 | Gew.% | Cr |
| 8 - 12 | Gew. % | W |
| 0,05 - 0,4 | Gew.% | jeweils eines oder mehrerer der Elemente der 4. und/oder 5. Nebengruppe des Periodensystems der Elemente; |
optional eines oder mehrere der Elemente Mn, Si, und N mit folgenden Anteilen:
| | | |
|---|---|---|
| 0 - 1,0 | Gew.% | Mn |
| 0,8 - 1,6 | Gew.% | Si |
| 0,1 - 0,35 | Gew.% | N; |
herstellungsbedingten Verunreinigungen;
Rest Kobalt.

2. Legierung nach Anspruch 1, **dadurch gekennzeichnet, dass** der Gesamtgehalt der Elemente der 4. und/oder 5. Nebengruppe des Periodensystems der Elemente ca. 0,05 bis ca. 0,4 Gew.% beträgt.

3. Legierung nach Anspruch 1 oder 2, **dadurch gekennzeichnet, dass** die Elemente der 4. und/oder 5. Nebengruppe des Periodensystems der Elemente ausgewählt sind aus Titan, Zirkon, Niob und Tantal.

4. Legierung nach Anspruch 3, **dadurch gekennzeichnet, dass** die die Legierung im Wesentlichen frei von den Elementen Eisen, Vanadium, Nickel, Molybdän und/oder Kohlenstoff ist.

## Claims

1. Dental casting alloy, in particular, for the production of dental prosthetic frameworks, comprising:
| | | |
|---|---|---|
| from 25 to 32 | wt% | of Cr, |
| from 8 to 12 | wt% | of W, |
| from 0.05 to 0.4 | wt%, | in each case, of one or more elements in sub-group IVa and/or sub-group Va of the periodic table of the elements; |
optionally one or more of the elements Mn, Si and N in the following fractions:
| | |
|---|---|
| from 0 to 1.0 | wt% of Mn, |
| from 0.8 to 1.6 | wt% of Si, |
| from 0.1 to 0.35 | wt% of N; |
production-related impurities;
the rest being cobalt.

2. Alloy as defined in claim 1, **characterized in that** the total content of sub-group IVa and/or sub-group Va elements of the periodic table of the elements is from approximately 0.05 to approximately 0.4 wt%.

3. Alloy as defined in claim 1 or 2, **characterized in that** the sub-group IVa and/or sub-group Va elements of the periodic table of the elements are selected from titanium, zirconium, niobium and tantalum.

4. Alloy as defined in claim 3, **characterized in that** the alloy is substantially free from the elements iron, vanadium, nickel, molybdenum and/or carbon.

## Revendications

1. Alliage de fonderie à usage dentaire, en particulier pour la fabrication d'ossatures de prothèses dentaires, ledit alliage étant constitué de :
25 à 32 % en poids de CR
8 à 12 % en poids de W
0,05 à 0,4 % en poids de un ou plusieurs des éléments des 4^{ième} et/ou 5^{ième} sous-groupes de la classification périodique des éléments ;
en option un ou plusieurs des éléments Mn, Si et N avec les pourcentages suivants :
0 à 1,0 % en poids de Mn
0,8 à 1,6 % en poids de Si
0,1 à 0,35 % en poids de N ;
des impuretés liées à la fabrication ;
le reste en cobalt.

2. Alliage selon la revendication 1, **caractérisé en ce que** la teneur totale en les éléments des 4^{ième} et/ou 5^{ième} sous-groupes de la classification périodique des éléments est d'environ 0,05 à environ 0,4% en poids.

3. Alliage selon la revendication 1 ou 2, **caractérisé en ce que** les éléments des 4^{ième} et/ou 5^{ième} sous-groupes de la classification périodique des éléments sont choisis parmi le titane, le zirconium, le niobium et le tantale.

4. Alliage selon la revendication 3, **caractérisé en ce que** l'alliage est essentiellement dépourvu des éléments fer, vanadium, nickel, molybdène et/ou carbone.
